# EUROPÄISCHE PATENTSCHRIFT

(11) **EP 2 116 473 B1**
(45) Veröffentlichungstag und Bekanntmachung des Hinweises auf die Patenterteilung: **17.02.2016**
(21) Anmeldenummer: 09005353.9
(22) Anmeldetag: 15.04.2009
(51) Int. Cl.: B65C 9/42, B65H 7/12, G01N 29/11, G01N 33/34

(54) **Ultraschallsensor**
Ultrasound sensor
Capteur d'ultrasons

(30) Priorität: 10.05.2008 DE 102008023185
(43) Veröffentlichungstag der Anmeldung: 11.11.2009
(73) Patentinhaber: Leuze electronic GmbH + Co. KG, 73277 Owen/Teck (DE)
(72) Erfinder: Patz, Jürgen, 72660 Beuren (DE); Merk, Thomas, 73240 Wendlingen (DE); Klaiber, Jörg, 71157 Hildrizhausen (DE)
(74) Vertreter: Ruckh, Rainer Gerhard

(56) Entgegenhaltungen:
- EP-A- 1 067 053
- EP-B- 0 167 010
- WO-A-2007/017663
- DE-A1- 3 633 556
- DE-A1- 3 939 387

## Beschreibung

Die Erfindung betrifft einen Ultraschallsensor.

Derartige Ultraschallsensoren werden zur Objektdetektion in unterschiedlichen Applikationen eingesetzt. Generell weist ein solcher Ultraschallsensor wenigstens einen Ultraschallwellen emittierenden Sender und einen Ultraschallwellen empfangenden Empfänger auf. Zur Generierung eines Objektfeststellungssignals werden in einer Auswerteeinheit die am Ausgang des Empfängers anstehenden Empfangssignale ausgewertet. Arbeitet der Ultraschallsensor nach dem Reflexionsprinzip, so wird in der Auswerteeinheit aus den Empfangssignalen der Anteil der vom Sender emittierten Ultraschallwellen, die von einem Objekt zurückreflektiert werden, ermittelt. Bei einem nach dem Transmissionsprinzip arbeitenden Ultraschallsensor wird in der Auswerteeinheit aus den Empfangssignalen der Anteil der vom Sender emittierten Ultraschallwellen ermittelt, die eine zu detektierende Objektstruktur durchdringen. Insbesondere können auf diese Weise mehrlagige Objektstrukturen erfasst werden.

Ein solcher Ultraschallsensor ist in der DE 199 21 217 A1 beschrieben. Dieser Ultraschallsensor dient zur Detektion von Etiketten auf Trägermaterialien.

Der dort beschriebene Ultraschallsensor ist in einem gabelförmigen Sensorgehäuse integriert, welches zwei parallel in Abstand zueinander verlaufende Gabelarme aufweist, wobei in einem ersten Gabelarm der Sender und in einem zweiten Gabelarm der Empfänger integriert ist. Der Zwischenraum zwischen den Gabelarmen bildet den Überwachungsbereich, innerhalb dessen die Objekte erfassbar sind.

Die zur Objektdetektion genutzten Ultraschallwellen treten aus einer Austrittsfläche aus dem ersten Gabelarm aus. Der die Objekte durchsetzende Teil der Ultraschallwellen, der direkt über eine Eintrittsfläche im zweiten Gabelarm zum Empfänger geführt ist, generiert im Empfänger ein Nutzsignal, anhand dessen die Objekte detektierbar sind. Jedoch treten bei derartigen Ultraschallsensoren Störsignale dadurch auf, dass ein Teil der Ultraschallwellen zwischen den Gabelarmen mehrfach reflektiert wird, wodurch sich stehende Ultraschallwellen zwischen den Gabelarmen bilden. Die durch derartige stehende Ultraschallwellen generierten Signalanteile im Empfänger bilden Störsignale, die die Objektdetektion verfälschen, das heißt die Nachweissicherheit des Ultraschallsensors reduzieren.

Die EP 0 167 010 B1 betrifft eine Vorrichtung zur Bestimmung des Flächengewichts von blattförmigem Material. Die Vorrichtung weist einen Ultraschallsensor mit einem Sender und Empfänger auf, welche beidseits des blattförmigen Materials in Abstand zu diesem angeordnet sind. Im Bereich der Auflage des blattförmigen Materials ist eine Platte mit einer Plattenöffnung vorgesehen, durch welche der Durchmesser der vom Sender emittierten Ultraschallwellen begrenzt wird.

Die DE 36 33 556 A1 betrifft eine Vorrichtung zur zerstörungsfreien Prüfung von Werkstoffen, zum Beispiel hochbeanspruchten Bauteilen aus faserverstärkten Kunststoffen und sonstigen Werkstoffen mit starker Ultraschallstreuung auf kleinste Fehler mittels Durchschallung mit Ultraschallwellen, wobei vor dem Empfänger-Prüfkopf eine Blende gesetzt wird, die die seitlich einfallenden Streusignale vom Empfänger-Prüfkopf fernhält, den momentan geprüften Bereich eines Bauteils wesentlich verkleinert und durch das vergrößerte Verhältnis von Fehlerfläche zu Prüfbereich das Nachweisvermögen für kleine Fehler verbessert. Die Blende weist eine Kegelform auf, wobei deren Spitze einem Sender-Prüfkopf zugeordnet ist.

Der Erfindung liegt die Aufgabe zugrunde, einen Ultraschallsensor mit hoher Nachweissicherheit bereitzustellen.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Der erfindungsgemäße Ultraschallsensor dient zur Erfassung von Objekten in einem Überwachungsbereich und umfasst einen Ultraschallwellen emittierenden Sender, einen Ultraschallwellen empfangenden Empfänger, eine Auswerteeinheit zur Generierung eines Objektfeststellungssignals in Abhängigkeit der Empfangssignale am Ausgang des Empfängers und ein zwei Gabelarme aufweisendes Sensorgehäuse. Die Gabelarme begrenzen den Überwachungsbereich. In einem ersten Gabelarm ist eine Austrittsfläche vorgesehen, durch welche die Ultraschallwellen in den Überwachungsbereich geführt sind. Im zweiten Gabelarm ist eine Eintrittsfläche vorgesehen, durch welche Ultraschallwellen aus dem Überwachungsbereich in das Sensorgehäuse geführt sind. An der Eintrittsfläche ist eine Blende vorgesehen. Durch das Blendenloch der Blende wird eine Begrenzung des Strahlquerschnitts der Ultraschallwellen erhalten. Die Stirnseite des Gabelarms, an welchem die Eintrittsfläche vorgesehen ist, weist einen konvexen Querschnitt auf, wobei die Blende auf der Stirnseite des Gabelarms dicht anliegt. Die Form der Blende ist an die Kontur des Gabelarms angepasst.

Durch die erfindungsgemäße Anbringung der empfangsseitigen Blende kann der Störeinfluss von sich zwischen den Gabelarmen ausbildenden stehenden Wellen signifikant reduziert werden, wodurch eine beträchtliche Erhöhung der Nachweisempfindlichkeit des Ultraschallsensors erzielt wird.

Diese Nachweisempfindlichkeit kann noch weiter dadurch gesteigert werden, indem auch sendeseitig eine Blende vorgesehen wird.

Die Unterdrückung stehender Wellen kann dadurch noch weiter gesteigert werden, dass die einander zugewandten Stirnseiten der Gabelarme, an welchen die Austrittsflächen und die Eintrittsflächen vorgesehen sind, einen konvex geformten Querschnitt aufweisen.

An den konvex geformten Stirnseiten der Gabelarme werden die Ultraschallwellen aus den Bereichen der Blendenlöcher der Blenden, welche auf den Stirnseiten der Gabelarme dicht aufliegen, wegreflektiert. Dadurch wird vermieden, dass an Komponenten des Sensorgehäuses mehrfach reflektierte Ultraschallwellen durch die Blendenlöcher der Blenden gelangen können.

Besonders vorteilhaft ist die oder jede Blende auf den jeweiligen Gabelarm aufrastbar oder aufschnappbar, wobei die oder jede Blende aus einem Blechteil besteht.

Die so ausgebildeten Blenden sind einerseits sehr kostengünstig herstellbar. Andererseits können die Blenden einfach und schnell am Sensorgehäuse montiert und auch wieder von diesem gelöst werden. Damit können insbesondere auch Blenden mit verschiedenen Blendenlöchern einfach am Sensorgehäuse ausgetauscht werden. Durch eine Variation der Blendenlöcher ist eine einfache Anpassung des Ultraschallsensors zur Detektion von Objekten mit verschiedenen Größen möglich. Insbesondere können durch Blenden mit sehr kleinen Blendenlöchern schmale Strahlquerschnitte der Ultraschallwellen erhalten werden, wodurch auch sehr kleine Objekte detektiert werden können.

Die oder jede Blende liegt auf der jeweiligen Stirnseite des Gabelarms dicht auf. Anschließend ist an die Stirnseite seitlich am jeweiligen Gabelarm ein eine Reinigungsöffnung bildender Zwischenraum zwischen Gabelarm und Blende vorgesehen.

Im Gegensatz zu den bisher üblichen Reinigungsöffnungen an den vorderen freien Enden der Gabelarme wird bei den erfindungsgemäß vorgesehen seitlichen Reinigungsöffnungen ein unerwünschtes Austreten von Ultraschallwellen über die Reinigungsöffnung und eine dadurch bedingte unerwünschte Reflexion dieser Ultraschallwellen an Maschinenteilen und dergleichen, vermieden. Störeinflüsse aufgrund derartiger Reflexionen von Ultraschallwellen können dadurch vermieden werden.

In einer vorteilhaften Ausgestaltung der Erfindung ist an wenigstens eine Blende eine Markierung zur Anzeige der Position des Blendenlochs vorgesehen. Als Markierungen sind an der der Eintrittsfläche zugeordneten Blende seitlich an dieser angeordnete Laschen vorgesehen.

Damit ist die Lage der Ultraschallwellen und somit auch die exakte Stelle, an welcher die Objektdetektion stattfindet, optisch hervorgehoben und für einen Benutzer gut sichtbar. Damit können im Überwachungsbereich zwischen den Gabelarmen geförderte Objekte gut ausgerichtet werden um diese sicher zu detektieren.

Die Erfindung wird im Folgenden anhand der Zeichnungen erläutert. Es zeigen:
- Figur 1:: Ultraschallsensor mit einem zwei Gabelarme aufweisenden Sensorgehäuse und zwei auf die Gabelarme aufgebrachten Blenden.
- Figur 2:: Ultraschallsensor gemäß Figur 1 mit einer gegenüber Figur 1 geänderten sendeseitigen Blende.
- Figur 3a-c:: Perspektivische Darstellung der Blenden für den Ultraschallsensor gemäß Figuren 1 und 2.
- Figur 4:: Erste Querschnittsdarstellung des Ultraschallsensors gemäß den Figuren 1 und 2.
- Figur 5:: Zweite Querschnittsdarstellung des Ultraschallsensor gemäß den Figuren 1 und 2.
- Figur 6:: Zeitabhängigkeit der sende- und empfangsseitigen Ultraschallwellen für den Ultraschallsensor gemäß Figuren 1, 2 ohne Blenden.
- Figur 7:: Zeitabhängigkeit der sende- und empfangsseitigen Ultraschallwellen für den Ultraschallsensor gemäß Figuren 1, 2 mit Blenden.

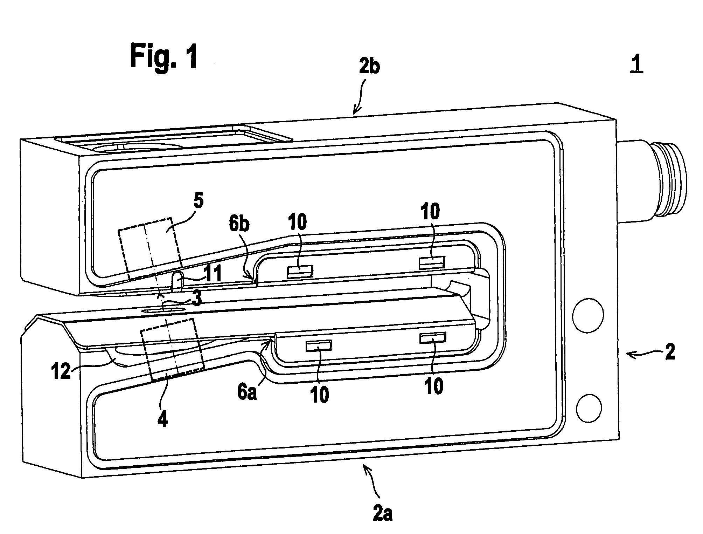

Figur 1 zeigt ein Ausführungsbeispiel eines Ultraschallsensors 1 zur Erfassung von Objekten. Der Ultraschallsensor 1 kann insbesondere zur Detektion von mehrlagigen Objektstrukturen eingesetzt werden. Beispielsweise können mit dem Ultraschallsensor 1 Etiketten auf Trägermaterialien detektiert werden. Weiterhin kann mit dem Ultraschallsensor 1 eine Doppelbogenkontrolle derart durchgeführt werden, dass Einfachbögen von Doppelbögen, das heißt zwei aufeinander liegenden Bögen, unterschieden werden können.

Die Komponenten des Ultraschallsensors 1 sind in einem gabelförmigen Sensorgehäuse 2 integriert. Das Sensorgehäuse 2 besteht vorzugsweise aus Kunststoff, alternativ kann das Sensorgehäuse 2 aus Metall bestehen.

Das Sensorgehäuse 2 ist gabelförmig ausgebildet und weist zwei parallel in Abstand zueinander verlaufende Gabelarme 2a, 2b auf. Der Ultraschallsensor 1 weist einen Ultraschallwellen 3 emittierenden Sender 4 und einen Ultraschallwellen 3 empfangenden Empfänger 5 auf. Im vorliegenden Fall emittiert der Sender 4 Ultraschallwellen 3 in Form von Sendepulspaketen mit definierten Pulsdauern und Pulspausen. Die Ultraschallwellen 3 sind in Figur 1 schematisch mit einem Pfeil gekennzeichnet. Der Sender 4 ist im ersten Gabelarm 2a schräg gestellt angeordnet, so dass die Strahlrichtung der Ultraschallwellen 3 in einem Neigungswinkel zur Horizontalen und damit zu den Längsachsen der Gabelarme 2a, 2b verläuft. Der Empfänger 5 ist im zweiten Gabelarm 2b mit einer entsprechenden Neigung angeordnet. Der Zwischenraum zwischen den Gabelarmen 2a, 2b bildet den Überwachungsbereich, innerhalb dessen Objekte erkannt werden.

Die Neigung der Strahlachse der Ultraschallwellen 3 verläuft in der von den Längsachsen der Gabelarme 2a, 2b aufgespannten Ebene und senkrecht zur Förderrichtung, in welcher die zu detektierenden Objekte durch den Zwischenraum zwischen den Gabelarmen 2a, 2b bewegt werden. Die Förderrichtung der Objekte verläuft senkrecht zur Zeichenebene. Durch diese Anordnung wird jeweils die Ausbildung von bei der Objektdetektion störenden stehenden Wellen reduziert.

In dem Sensorgehäuse 2 ist eine nicht gesondert dargestellte Auswerteeinheit angeordnet, die einerseits zur Ansteuerung des Senders 4 und andererseits zur Auswertung der Empfangssignale des Empfängers 5 dient. Die Objektdetektion erfolgt dabei nach dem Transmissionsprinzip, indem im Empfänger 5 der ein Objekt durchsetzende Teil der Ultraschallwellen 3 registriert und in der Auswerteeinheit ausgewertet wird. In Abhängigkeit der Empfangssignale wird in der Auswerteeinheit ein Objektfeststellungssignal generiert. Insbesondere kann in der Auswerteeinheit durch eine Schwellwertbewertung ein binäres Schaltsignal generiert werden. Die beiden Schaltzustände des Schaltsignals geben im einfachsten Fall an, ob ein Objekt vorhanden ist oder nicht. Für den Fall, dass die Objekte von auf Trägermaterialien angeordneten Etiketten gebildet sind, geben die Schaltzustände an, ob eine Etikette auf dem Trägermaterial vorhanden ist oder nicht. Für den Fall, dass mit dem Ultraschallsensor 1 eine Doppelbogenkontrolle durchgeführt wird, geben die Schaltzustände des Schaltsignals an, ob ein Einfach- oder Doppelbogen vorliegt.

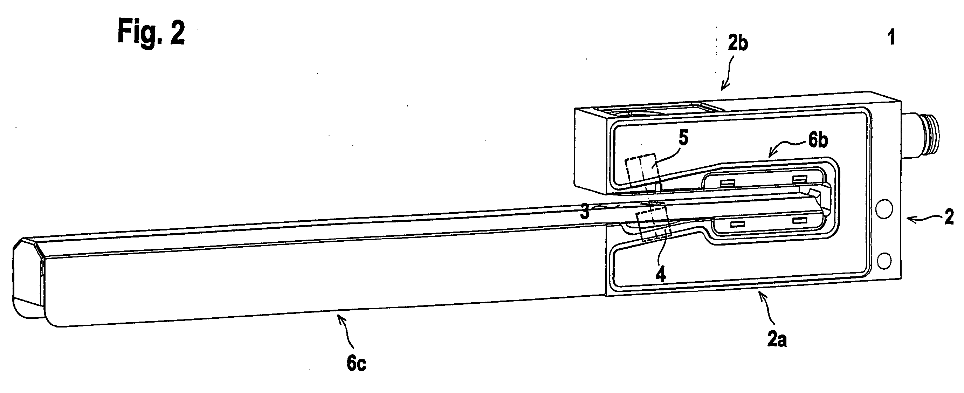

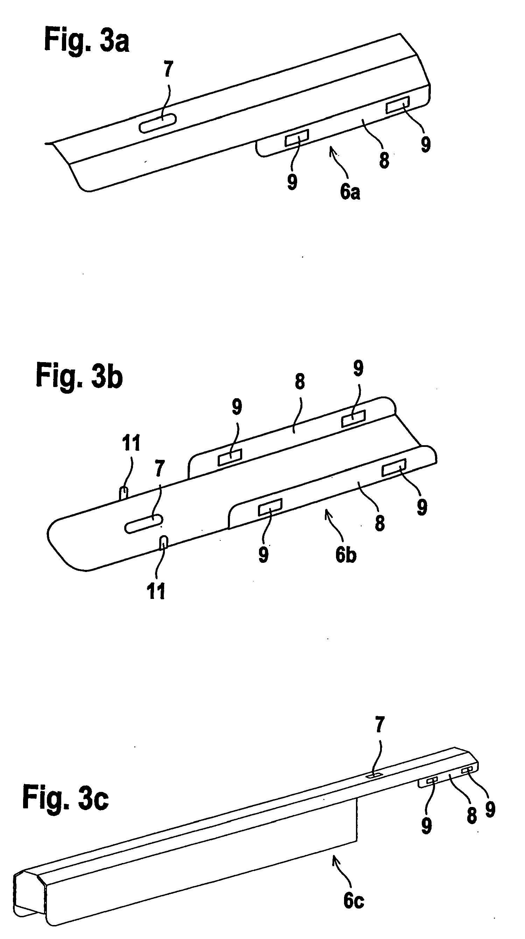

Wie aus Figur 1 ersichtlich, ist am ersten Gabelarm 2a eine erste Blende 6a und am zweiten Gabelarm 2b eine zweite Blende 6b angeordnet. Figur 2 zeigt den Sensor gemäß Figur 1 mit einer dritten Blende 6c am ersten Gabelarm 2a, wobei am zweiten Gabelarm 2b dieselbe Blende 6b wie im Ausführungsbeispiel gemäß Figur 1 angeordnet ist. Die drei Blenden 6a, 6b, 6c sind in den Figuren 3a- 3c in perspektivischen Einzeldarstellungen dargestellt.

Die Blenden 6a, 6b, 6c bestehen jeweils aus Blechteilen und können durch Aufrasten oder Aufschnappen am jeweiligen Gabelarm 2, 2b befestigt und bei Bedarf von diesem wieder gelöst werden, beispielsweise um die jeweilige Blende 6a, 6b, 6c durch eine andere Blende 6a, 6b, 6c zu ersetzen.

Wie aus den Figuren 3a - 3c ersichtlich, weist jede der Blenden 6a, 6b, 6c ein Blendenloch 7 auf. Bei einer senderseitigen Blende 6a, 6c ist das Blendenloch 7 so angeordnet, dass bei auf dem Gabelarm 2a aufgesetzter Blende 6a, 6c das Blendenloch 7 direkt vor der Austrittsfläche, an welche die Ultraschallwellen 3 aus dem Gabelarm 2a austreten, angeordnet. Durch das Blendenloch 7 wird eine Begrenzung des Strahlquerschnitts der Ultraschallwellen 3 erhalten. Zur Variation des Strahlquerschnitts können mehrere Blenden 6a, 6b, 6c gleichen Typs, jedoch mit Blendenlöcher 7 verschiedener Größen und Geometrien verwendet werden.

Bei der empfangsseitigen Blende 6b ist das Blendenloch 7 so angeordnet, dass bei auf dem Gabelarm 2b aufgebrachter Blende 6b das Blendenloch 7 direkt vor der Eintrittsfläche, über welche Ultraschallwellen 3 aus dem Überwachungsbereich in den Gabelarm 2b geführt werden, sitzt. Durch die Blende 6b wird erreicht, dass nur ein eng begrenzter Anteil der Ultraschallwellen 3 auf den Empfänger 5 trifft. Auch für einen Typ einer empfangesseitigen Blende 6a, 6b, 6c können Varianten mit Blendenlöchern 7 verschiedener Größen und Geometrien vorgesehen sein. Überraschenderweise hat sich herausgestellt, dass allein durch das Vorsehen der empfangsseitigen Blende 6a, 6b, 6c der Anteil an stehenden Wellen, die durch Mehrfachreflexionen der Ultraschallwellen 3 an den Wänden der Gabelarme 2a, 2b entstehen, erheblich reduziert werden kann, wodurch die Nachweissicherheit des Ultraschallsensors erhöht wird. Das zusätzliche Vorsehen einer senderseitigen Blende 6a, 6b, 6c erhöht diesen Effekt noch.

Wie aus den Figuren 1, 2 sowie 3a - 3c ersichtlich, weist jede Blende 6a - 6c an ihren zwei längsseitigen Rändern jeweils eine Befestigungslasche 8 auf, welche identisch ausgebildet sind. In jeder Befestigungslasche 8 sind Aussparungen 9 vorgesehen. Durch Einrasten der Aussparungen 9 in von den Wänden der Gabelarme 2a, 2b hervorstehenden Vorsprünge 10, welche Rastaufnahmen bilden, wird die Blende 6a - 6c am jeweiligen Gabelarm 2a, 2b fixiert.

Wie aus der Figur 1 und insbesondere aus Figur 3a ersichtlich, sind an der senderseitigen Blende 6a seitlich Markierungen in Form von Laschen 11 vorgesehen, die die Position des Blendenlochs 7 markieren. Bei auf dem Gabelarm 2a aufgerasteter Blende 6a zeigen die Laschen 11 gut sichtbar die Lage der Strahlachse der Ultraschallwellen 3 und damit den genauen Detektionsort an, so dass Objekte genau auf diesen ausgerichtet werden können.

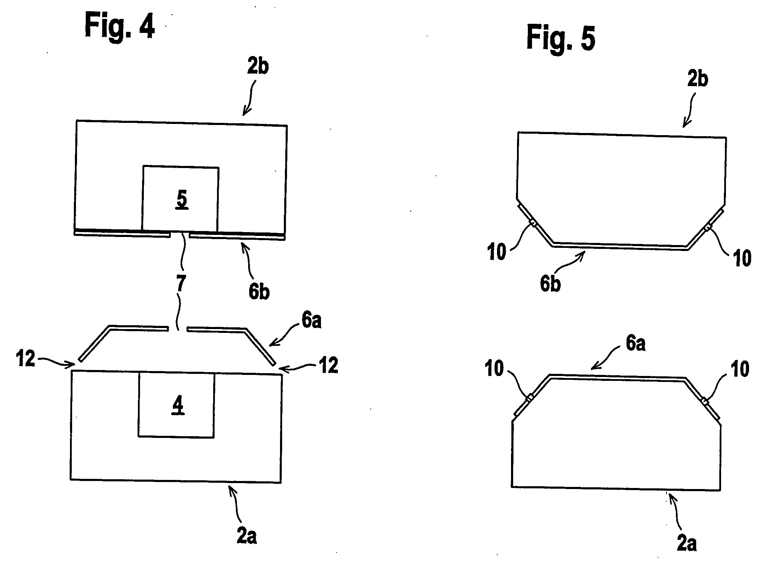

Figur 4 zeigt einen Querschnitt durch den Ultraschallsensor 1 gemäß Figur 1 in der Ebene A, während Figur 5 einen Querschnitt durch den Ultraschallsensor 1 gemäß Figur 1 in der Ebene B zeigt. Wie aus den Figuren 1, 4, 5 ersichtlich, sind die Konturen der Blenden 6a, 6b an die Konturen der einander gegenüberliegenden Stirnseiten der Gabelarme 2a, 2b angepasst. Damit liegen die Blenden 6a, 6b, wie insbesondere aus Figur 5 ersichtlich, dicht auf den Stirnseiten der Gabelarme 2a, 2b auf. Wie aus Figur 5 ersichtlich, weisen die Stirnseiten der Gabelarme 2a, 2b in diesem Bereich konvex ausgebildete Stirnflächen auf, wodurch Mehrfachreflexionen von Ultraschallwellen 3 in diesem Bereich vermieden werden, da die Ultraschallwellen 3 an den Schrägflächen seitlich weg reflektiert werden. Wie aus Figur 1 ersichtlich, liegen die Blenden 6a, 6b auch im Bereich der vorderen freien Enden der Gabelarme 2a, 2b dicht auf deren Stirnseiten der Gabelarme 2a, 2b auf und schließen bündig mit den Vorderenden der Gabelarme 2a, 2b ab. Damit bildet die Blende 6a im Bereich des vorderen Endes des Gabelarms 2a einen dichten Abschluss, der verhindert, dass dort Ultraschallwellen 3 austreten können. Dadurch werden Rückreflexionen von dort austretenden Ultraschallwellen 3 an Maschinenteilen und dergleichen zurück in den Detektionsbereich vermieden.

Wie aus Figur 1 ersichtlich, ist die Stirnseite des Gabelarms 2a im Bereich des Senders 4 nach unten verlaufend abgeschrägt, wodurch, wie aus Figuren 1 und 4 ersichtlich, einerseits seitliche Reinigungsöffnungen 12 und andererseits ein als Resonanzraum dienender Zwischenraum zwischen der Stirnseite des Gabelarms 2a und der Blende 6a - 6c entsteht.

Über die Reinigungsöffnungen kann der Zwischenraum zwischen Gabelarm 2a und Blende 6a - 6c leicht gereinigt werden. Zudem stören dort seitlich austretende Ultraschallwellen 3 das Detektionsverhalten des Ultraschallsensors 1 nicht.

Die Blende 6a auf dem Gabelarm 2a bildet eine Auflagefläche für die zu detektierenden Objekte. Diese auf die Ausdehnung des Gabelarms 2a begrenzte Auflagefläche ist ausreichend, wenn die zu detektierenden Objekte eine geringe Ausdehnung quer zur Förderrichtung aufweisen.

Für den Fall, dass die Objekte eine größere Breite aufweisen, wird anstelle der in Figur 1 dargestellten Blende 6a die in Figur 2 dargestellte Blende 6c eingesetzt. Die Blende 6c weist in dem Bereich, in welchem sie auf dem Gabelarm 2a aufliegt, dieselbe Form wie die Blende 6a auf. Zusätzlich weist die Blende 6c ein weit über den Gabelarm 2a hervorstehendes Segment auf, mittels dessen die Auflagefläche für die Objekte erheblich vergrößert wird.

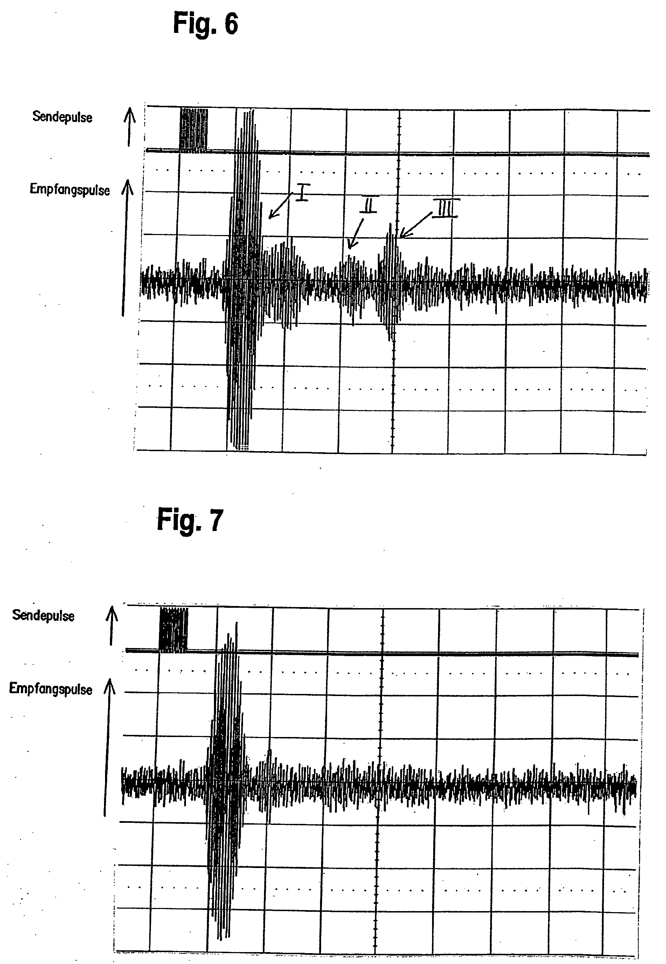

Figur 6 zeigt ein Zeitdiagramm eines (mit I bezeichneten) Sendepulspakets, das vom Sender 4 des Ultraschallsensors 1 gemäß Figur 1 beziehungsweise 2 emittiert wird. Zudem zeigt Figur 6 die auf das emittierte Sendepulspaket am Empfänger 5 registrierten Empfangspulse und zwar für den Fall, dass vor dem Empfänger 5 keine Blende 6b angeordnet ist. Wie aus Figur 6 ersichtlich, wird auf das Aussenden eines Sendepulspakets nicht nur das vom Objekt stammende Nutzsignal, das in Figur 6 mit I bezeichnete Empfangspulspaket empfangen. Vielmehr werden am Empfänger 5 auch mehrere (mit II und III bezeichnete) Störsignalpulse empfangen, die durch Mehrfachreflexionen entstehen. Diese überlagern sich mit folgenden Nutzsignalen, die für weitere, folgende Sendepulspakete erhalten werden (welche in Figur 6 der Übersichtlichkeit halber nicht dargestellt sind), und führen so zu einer Verfälschung der Messergebnisse.

Figur 7 zeigt dasselbe Sendepulspaket des Ultraschallsensors 1 wie in Figur 6 und die zugehörigen Empfangspulse für den Fall, dass vor dem Empfänger 5 die Blende 6b angeordnet ist. Wie aus Figur 7 ersichtlich, sind durch den Einsatz der Blende 6b die in Figur 6 noch vorhandenen Störimpulse II, III vollständig eliminiert, da durch den Einsatz der Blende 6b Mehrfachreflexionen der Ultraschallwellen 3 zwischen den Gabelarmen 2a, 2b vermieden werden.

## Patentansprüche

1. Ultraschallsensor (1) zur Erfassung von Objekten in einem Überwachungsbereich, mit einem Ultraschallwellen (3) emittierenden Sender (4), einem Ultraschallwellen (3) empfangenden Empfänger (5), einer Auswerteeinheit zur Generierung eines Objektfeststellungssignals in Abhängigkeit der Empfangssignale am Ausgang des Empfängers (5), und mit einem zwei Gabelarme aufweisenden Sensorgehäuse (2), wobei die Gabelarme den Überwachungsbereich begrenzen, und wobei in einem ersten Gabelarm eine Austrittsfläche vorgesehen ist, durch welche die Ultraschallwellen (3) in den Überwachungsbereich geführt sind, und im zweiten Gabelarm eine Eintrittsfläche vorgesehen ist, durch welche Ultraschallwellen (3) aus dem Überwachungsbereich in das Sensorgehäuse (2) geführt sind, **dadurch gekennzeichnet, dass** an der Eintrittsfläche eine Blende (6a - 6c) vorgesehen ist, durch deren Blendenloch (7) eine Begrenzung des Strahlquerschnitts der Ultraschallwellen (3) erhalten wird, wobei die Stirnseite des Gabelarms (2b), an welchem die Eintrittsfläche vorgesehen ist, einen konvex geformten Querschnitt aufweist, wobei die Blende (6a bis 6c) auf der Stirnseite des Gabelarms dicht aufliegt, und wobei die Form der Blende (6a - 6c) an die Kontur des Gabelarms (2a, 2b) angepasst ist.

2. Ultraschallsensor nach Anspruch 1, **dadurch gekennzeichnet, dass** an der Austrittsfläche eine Blende (6a - 6c) vorgesehen ist, wobei die Form der Blende (6a - 6c) an die Kontur des Gabelarms (2a, 2b) angepasst ist, und wobei die Stirnseite des Gabelarms (2a), an welchem die Austrittsflächen vorgesehen sind, einen konvex geformten Querschnitt aufweist, wobei die oder jede Blende (6a - 6c) auf der Stirnseite des Gabelarms (2a) dicht aufliegt.

3. Ultraschallsensor nach einem der Ansprüche 1 oder 2, **dadurch gekennzeichnet, dass** die oder jede Blende (6a - 6c) auf den jeweiligen Gabelarm (2a, 2b) aufrastbar oder aufschnappbar ist.

4. Ultraschallsensor nach Anspruch 3, **dadurch gekennzeichnet, dass** die oder jede Blende (6a - 6c) aus einem Blechteil besteht.

5. Ultraschallsensor nach einem der Ansprüche1 bis 4, **dadurch gekennzeichnet, dass** anschließend an die Stirnseite seitlich am jeweiligen Gabelarm (2a, 2b) ein eine Reinigungsöffnung bildender Zwischenraum zwischen Gabelarm (2a, 2b) und Blende (6a - 6c) vorgesehen ist.

6. Ultraschallsensor nach einem der Ansprüche 3 bis 5, **dadurch gekennzeichnet, dass** an gegenüberliegenden Längsseiten einer Blende (6a - 6c) Befestigungslaschen (8) vorgesehen sind, welche an Rastaufnahmen des jeweiligen Gabelarms (2a, 2b) fixierbar sind.

7. Ultraschallsensor nach einem der Ansprüche 1 bis 6, **dadurch gekennzeichnet, dass** an den jeweiligen Gabelarm (2a, 2b) verschiedene Blenden (6a - 6c) mit unterschiedlich ausgebildeten Blendenlöchern (7) fixierbar sind.

8. Ultraschallsensor nach Anspruch 7, **dadurch gekennzeichnet, dass** an wenigstens einer Blende (6a - 6c) eine Markierung zur Anzeige der Position des Blendenlochs (7) vorgesehen ist.

9. Ultraschallsensor nach Anspruch 8, **dadurch gekennzeichnet, dass** als Markierungen an der der Eintrittsfläche zugeordneten Blende (6a - 6c) seitlich an dieser angeordnete Laschen (11) vorgesehen sind.

10. Ultraschallsensor nach einem der Ansprüche 2 bis 9, **dadurch gekennzeichnet, dass** die der Austrittsfläche zugeordnete Blende (6a - 6c) eine Auflagefläche für die zu detektierenden Objekte bildet.

11. Ultraschallsensor nach Anspruch 10, **dadurch gekennzeichnet, dass** die Blende (6a - 6c) über das freie, längsseitige Ende des Gabelarms (2a, 2b) hervorsteht.

12. Ultraschallsensor nach einem der Ansprüche 1 bis 11, **dadurch gekennzeichnet, dass** die zu detektierenden Objekte in einer quer zu den Längsachsen der Gabelarme (2a, 2b) verlaufenden Förderrichtung gefördert werden.

13. Ultraschallsensor nach einem der Ansprüche 1 bis 12, **dadurch gekennzeichnet, dass** die Objekte von auf einem Trägermaterial angeordneten Etiketten gebildet sind.

## Claims

1. Ultrasonic sensor (1) for detecting objects in a monitoring region, comprising an emitter (4) emitting ultrasonic waves (3), a receiver (5) receiving ultrasonic waves (3), an evaluating unit for generating an object detection signal in dependence on the received signals at the output of the receiver (5), and a sensor housing (2) having two fork arms, wherein the fork arms bound the monitoring region and wherein provided in a first fork arm is an exit surface through which the ultrasonic waves (3) are guided into the monitoring region and provided in the second fork arm is an entry surface through which ultrasonic waves (3) are led from the monitoring region into the sensor housing (2), **characterised in that** provided at the entry surface is a screen (6a - 6c), by the screen opening (7) of which a bounding of the beam cross-section of the ultrasonic waves (3) is attained, wherein the face of the fork arm (2b) at which the entry surface is provided has a convexly formed cross-section, wherein the screen (6a - 6c) rests tightly on the face of the fork arm and wherein the shape of the screen (6a - 6c) is matched to the contour of the fork arm (2a, 2b).

2. Ultrasonic sensor according to claim 1, **characterised in that** a screen (6a - 6c) is provided at the exit surface, wherein the shape of the screen (6a - 6c) is matched to the contour of the fork arm (2a, 2b) and wherein the face of the fork arm (2a) at which the exit surfaces are provided has a convexly shaped cross-section, wherein the or each screen (6a - 6c) tightly rests on the face of the fork arm (2a).

3. Ultrasonic sensor according to one of claims claim 1 and 2, **characterised in that** the or each screen (6a - 6c) can be detented or snapped into place on the respective fork arm (2a, 2b).

4. Ultrasonic sensor according to claim 3, **characterised in that** the or each screen (6a - 6c) consists of a sheet metal part.

5. Ultrasonic sensor according to any one of claims 1 to 4, **characterised in that** an intermediate space forming a cleaning opening between fork arm (2a, 2b) and screen (6a - 6c) is provided in connection with the face laterally at the respective fork arm (2a, 2b).

6. Ultrasonic sensor according to any one of claims 3 to 5, **characterised in that** fastening straps (8) fixable to detent mounts of the respective fork arm (2a, 2b) are provided at opposite longitudinal sides of a screen (6a - 6c).

7. Ultrasonic sensor according to any one of claims 1 to 6, **characterised in that** different screens (6a - 6c) with differently constructed screen openings (7) are fixable to the respective fork arm (2a, 2b).

8. Ultrasonic sensor according to claim 7, **characterised in that** a marking for indicating the position of the screen opening (7) is provided at at least one screen (6a - 6c).

9. Ultrasonic sensor according to claim 8, **characterised in that** provided as markings at the screen (6a - 6c) associated with the entry surface are straps (11) laterally arranged thereat.

10. Ultrasonic sensor according to any one of claims 2 to 9, **characterised in that** the screen (6a - 6c) associated with the exit surface forms a rest surface for the objects to be detected.

11. Ultrasonic sensor according to claim 10, **characterised in that** the screen (6a - 6c) protrudes beyond the free, longitudinal-side end of the fork arm (2a, 2b).

12. Ultrasonic sensor according to any one of claims 1 to 11, **characterised in that** the objects to be detected are conveyed in a conveying direction extending transversely to the longitudinal axes of the fork arms (2a, 2b).

13. Ultrasonic sensor according to any one of claims 1 to 12, **characterised in that** the objects are formed by labels arranged on a carrier material.

## Revendications

1. Capteur d'ultrasons (1) pour la détection d'objets dans une zone de surveillance, comprenant un émetteur (4) émettant des ondes ultrasonores (3), un récepteur (5) recevant des ondes ultrasonores (3), une unité d'évaluation pour générer un signal de détection d'objet en fonction des signaux de réception à la sortie du récepteur (5), et comprenant un boîtier de capteur (2) présentant deux bras de fourche, les bras de fourche délimitant la zone de surveillance, une surface de sortie étant prévue dans un premier bras de fourche, à travers laquelle les ondes ultrasonores (3) sont guidées dans la zone de surveillance, et une surface d'entrée étant prévue dans le deuxième bras de fourche, à travers laquelle les ondes ultrasonores (3) sont guidées de la zone de surveillance dans le boîtier de capteur (2), **caractérisé en ce qu'**un cache (6a - 6c) est prévu sur la surface d'entrée, dont l'orifice de cache (7) produit une limitation de la section du faisceau des ondes ultrasonores (3), la face frontale du bras de fourche (2b) sur lequel la surface d'entrée est prévue présentant une section transversale de forme convexe, le cache (6a à 6c) étant étroitement appliqué sur la face frontale du bras de fourche et la forme du cache (6a - 6c) étant adaptée au contour du bras de fourche (2a, 2b).

2. Capteur d'ultrasons selon la revendication 1, **caractérisé en ce qu'**un cache (6a - 6c) est prévu sur la surface de sortie, la forme du cache (6a - 6c) étant adaptée au contour du bras de fourche (2a, 2b), et la face frontale du bras de fourche (2a) sur lequel les surfaces de sortie sont prévues présentant une section transversale de forme convexe, le ou chaque cache (6a - 6c) étant étroitement appliqué sur la face frontale du bras de fourche (2a).

3. Capteur d'ultrasons selon l'une des revendications 1 ou 2, **caractérisé en ce que** le ou chaque cache (6a - 6c) est encliquetable ou clipsable sur le bras de fourche (2a, 2b) respectif.

4. Capteur d'ultrasons selon la revendication 3, **caractérisé en ce que** le ou chaque cache (6a - 6c) est constitué d'une pièce en tôle.

5. Capteur d'ultrasons selon l'une des revendications 1 à 4, **caractérisé en ce qu'**un espace formant une ouverture de nettoyage, se raccordant à la face frontale latéralement sur le bras de fourche (2a, 2b) respectif, est prévu entre le bras de fourche (2a, 2b) et le cache (6a - 6c).

6. Capteur d'ultrasons selon l'une des revendications 3 à 5, **caractérisé en ce que** sur les côtés longitudinaux opposés d'un cache (6a - 6c) sont prévues des pattes de fixation (8) qui peuvent être fixées à des logements d'encliquetage du bras de fourche (2a, 2b) respectif.

7. Capteur d'ultrasons selon l'une des revendications 1 à 6, **caractérisé en ce que** différents caches (6a - 6c) avec des orifices de cache (7) de forme différente peuvent être fixés au bras de fourche (2a, 2b) respectif.

8. Capteur d'ultrasons selon la revendication 7, **caractérisé en ce qu'**un repère pour indiquer la position de l'orifice de cache (7) est prévu sur au moins un cache (6a - 6c).

9. Capteur d'ultrasons selon la revendication 8, **caractérisé en ce que** des pattes (11) disposées latéralement sur le cache (6a - 6c) sont prévues comme repères sur le cache (6a - 6c) associé à la surface d'entrée.

10. Capteur d'ultrasons selon l'une des revendications 2 à 9, **caractérisé en ce que** le cache (6a - 6c) associé à la surface de sortie forme une surface d'appui pour les objets à détecter.

11. Capteur d'ultrasons selon la revendication 10, **caractérisé en ce que** le cache (6a - 6c) fait saillie de l'extrémité libre, du côté longitudinal du bras de fourche (2a, 2b).

12. Capteur d'ultrasons selon l'une des revendications 1 à 11, **caractérisé en ce que** les objets à détecter sont transportés dans un dispositif de transport s'étendant transversalement aux axes longitudinaux des bras de fourche (2a, 2b).

13. Capteur d'ultrasons selon l'une des revendications 1 à 12, **caractérisé en ce que** les objets sont formés par des étiquettes disposées sur un matériau de support.
